# EUROPEAN PATENT APPLICATION

(11) **EP 4 803 634 A1**
(43) Date of publication of application: **09.09.2026**
(21) Application number: 25161256.0
(22) Date of filing: 03.03.2025
(51) Int. Cl.: C12Q 1/18, C12Q 1/06

(54) **METHOD AND APPARATUS FOR ANTIBIOTIC SUSCEPTIBILITY TESTING OF A BACTERIAL SAMPLE**

(71) Applicant: Technische Universität München, in Vertretung des Freistaates Bayern, 80333 München (DE)
(72) Inventor: Sabersky-Müssigbrodt, Henning, 80469 München (DE); Hayden, Oliver, 85368 Moosburg (DE); Wali, Sarah, 81373 München (DE)
(74) Representative: Lucke, Andreas

(57) **Abstract**

Disclosed herein is a method, a computer program and an apparatus for antibiotic susceptibility testing of a bacterial sample. The method comprises determining a size of an inhibition zone that is formed when the sample is applied to a substrate and the sample on the substrate is exposed to an antibiotic; determining a concentration measure characterizing a sample concentration of bacteria in the sample; and assessing a susceptibility of the sample to said antibiotic using a predictive model configured to relate, based on the concentration measure, a size of the inhibition zone associated with the sample concentration to a size of the inhibition zone associated with a reference concentration of bacteria and/or vice-versa.

## Description

### FIELD OF THE DISCLOSURE

The present disclosure is in the field of medical diagnostics. In particular, the present disclosure relates to a method, a computer program and an apparatus for antibiotic susceptibility testing of bacterial samples.

### BACKGROUND

The effectiveness of antibiotics against bacterial infections such as urinary tract infections is conventionally determined by disk diffusion tests. These tests measure the size of an inhibition zone that is formed around an antibiotic-impregnated paper disk placed on a bacterial culture on an agar substrate. The size of this inhibition zone is then compared to standardized reference tables of threshold values as for example published by institutions such as the European Committee on Antimicrobial Susceptibility Testing (EUCAST) or the Clinical and Laboratory Standards Institute (CLSI) to classify the bacteria as either susceptible or resistant to the antibiotic. Such traditional disk diffusion tests, however, involve multiple specialized processing steps including pathogen isolation and suspension to a standardized concentration (typically to the 0.5 McFarland standard). These processing steps not only require specialized equipment as well as trained professionals but are also render these tests time consuming, typically resulting in processing times on the order of 36-48 hours.

To eliminate the need for pathogen isolation, direct susceptibility testing methods have been developed in recent years. These methods perform antibiotic susceptibility testing directly on patient specimens such as urine or sputum rather than on isolated bacterial samples with defined bacterial concentration, see for example L. Coorevits et al. Eur J Clin Microbiol Infect Dis (2015) 34:1207-1212 and R. C. She, Clinical Microbiology Newsletter 41:8, 2019*.* While these methods offer substantial benefits in terms of processing times, they often face challenges related to the accuracy and reliability of their results. As a result, these methods are prone to misclassifying bacteria as susceptible or resistant, which may lead to inefficient therapies and improper patient care.

It is thus an object of the present disclosure to provide means for antibiotic susceptibility testing that allow for accurately and reliably assessing antibiotic susceptibility while reducing diagnostic processing time.

This object is met by a method for antibiotic susceptibility testing of a bacterial sample, a computer program and an apparatus for antibiotic susceptibility testing of a bacterial sample as set out in the independent claims. Examples thereof are detailed in the dependent claims.

Any feature or combination(s) of features described herein with regard to the method, the computer program or the apparatus according to the present disclosure may also be present for any other one of the method, the computer program and the apparatus according to the present disclosure and vice-versa. For the sake of brevity and to avoid unnecessary repetition, certain feature(s) or combination(s) of features may therefore only be described once with regard to either the method, the computer program or the apparatus, but may likewise apply to any other one of the method, the computer program and the apparatus.

According to a first aspect, the present disclosure provides a method for antibiotic susceptibility testing of a bacterial sample. The method comprises determining a size of an inhibition zone that is formed when the sample is applied to a substrate and the sample on the substrate is exposed to an antibiotic. The method further comprises determining a concentration measure characterizing a sample concentration of bacteria in the sample. A susceptibility of the sample to the antibiotic is assessed using a predictive model, wherein the predictive model is configured to relate, based on the concentration measure, a size of the inhibition zone associated with the sample concentration to a size of the inhibition zone associated with a reference concentration of bacteria and/or vice-versa.

The method may be computer-implemented at least in part or in its entirety. The method may be executed at least in part or in its entirety using (and in particular by) the apparatus according to the present disclosure (e.g., the controller thereof) and/or the computer program according to the present disclosure.

The method may comprise one or both of applying the sample, e.g., in its entirety or an aliquot (part) thereof, to the substrate and exposing the sample on the substrate to the antibiotic. In other examples, the sample (e.g., in its entirety or an aliquot thereof) may be applied to the substrate and/or exposed to the antibiotic prior to execution of the method according to the present disclosure. For example, the sample may be provided after having been applied to the substrate and/or exposed to the antibiotic. In another example, only an image (or other recording) of the sample on the substrate after exposure to the antibiotic may be provided (and the method may, e.g., not comprise any handling of the sample at all).

When the sample on the substrate is exposed to the antibiotic (e.g., locally at an application site such as a particular position or in a particular area), the antibiotic may diffuse into the sample and/or the substrate. The antibiotic may be configured to inhibit growth of and/or kill bacteria, which may result in the formation of an inhibition zone at and/or around the application site. As used herein, the term inhibition zone may refer to a zone or area of reduced concentration of bacteria, in particular a zone or area that is free or substantially free from bacteria. The inhibition zone may for example be defined (and determined) following a standard such as the CLSI or EUCAST recommendations, see, e.g., European committee on antimicrobial susceptibility testing (EUCAST), Breakpoint Tables for Interpretation of MICs and Zone Diameters. Version 14.0 (2024*)* and Clinical and Laboratory Standards Institute, CLSI M100 - Performance Standards for Antimicrobial Susceptibility Testing, 34th Edition (2024*).*

The size of the inhibition zone may for example be determined (e.g., measured) from one or more images, for example camera images and/or microscopic images, of the sample on the substrate. In other examples, the size of the inhibition zone may, e.g., be determined directly on the sample (on the substrate), for example by visual inspection. The size of the inhibition zone as used herein may refer to any physical dimension or extent, in particular a diameter, a radius, a width and/or a cross-sectional area, of the inhibition zone (e.g., measured in a plane parallel to a top surface of the substrate).

The determined concentration measure characterizes (e.g., is indicative of, quantifies or allows for quantifying) a concentration of bacteria in the sample (referred to as the "sample concentration" herein). The sample concentration characterized by the concentration measure may for example be the concentration of bacteria in the sample (or an aliquot thereof) prior to applying the sample to the substrate, after applying the sample to the substrate (and prior to exposing the sample to the antibiotic) or after exposing the sample to the antibiotic (e.g., at the same time when determining the size of the inhibition zone). The concentration measure must, however, not necessarily be determined at the respective point in time and/or from the same (first) aliquot of the sample applied to the substrate but may also be determined at a different point in time (e.g., following one or more processing steps such as for example incubating the sample or an aliquot thereof) and/or from a different (second) aliquot of the sample. The concentration measure may, for example, be or quantify a concentration of bacteria at said different point in time (e.g., following said one or more processing steps) and/or in said second aliquot. The concentration of bacteria at said different point in time and/or in said second aliquot may depend on (e.g., be proportional to or in another functional relationship with) the sample concentration, thus characterizing the sample concentration.

The susceptibility of the sample to the antibiotic that the sample is exposed to is assessed based on the determined size of the inhibition zone. Assessing the susceptibility may for example comprise classifying and/or quantifying the susceptibility of the sample, for example by classifying the sample as "susceptible" or "resistant" (or, optionally, more finely divided categories, e.g., one or more intermediate categories such as "partially susceptible" or "partially resistant").

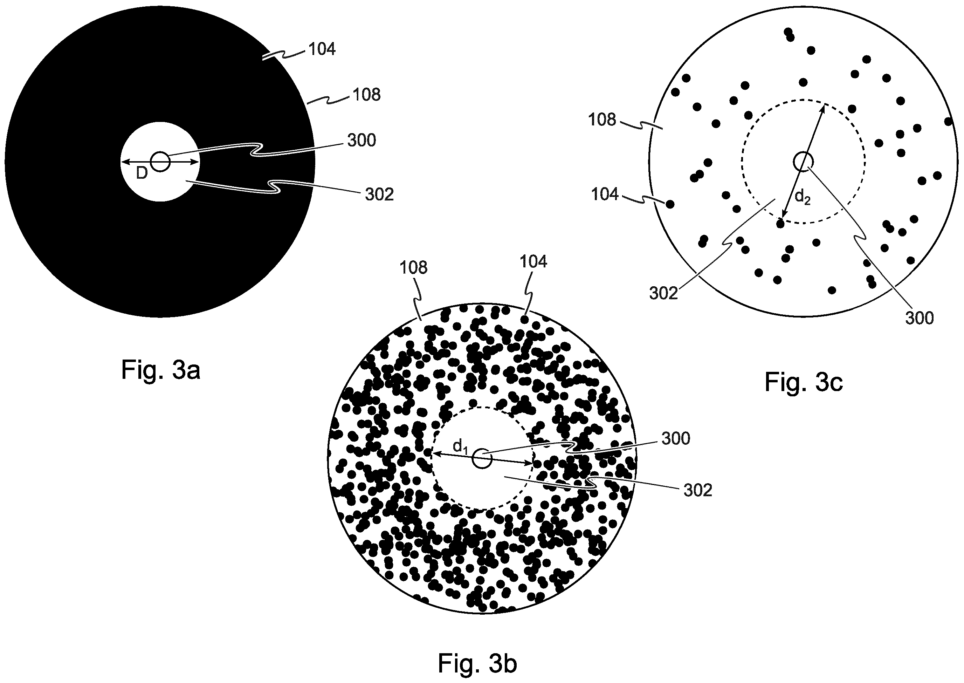

The present inventors have observed (and studied systematically) that the concentration of bacteria in the sample may crucially affect the size of the inhibition zone, e.g., as detailed below with reference to Figs. 3a and 3b. For example, a lower concentration may lead to a larger inhibition zone whereas a higher concentration may lead to a smaller inhibition zone. This may result in misclassification of resistant bacteria (of, e.g., low concentration) as susceptible or susceptible bacteria (of, e.g., high concentration) as resistant. As such, the accuracy and reliability of the assessed susceptibility may be improved significantly by additionally taking into account the determined concentration measure, i.e., assessing the susceptibility of the sample to the antibiotic that based on both the determined size of the inhibition zone and the determined concentration measure.

For this, the susceptibility of the sample to the antibiotic is assessed using a predictive model that relates (e.g., maps, converts or associates) a size of the inhibition zone associated with (for example at, e.g., determined at or expected at) the sample concentration to a size of the inhibition zone associated with (for example at, e.g., determined at or expected at) a reference concentration and/or vice-versa (i.e., additionally or alternatively, relates a size of the inhibition zone associated with the reference concentration to a size of the inhibition zone associated with the sample concentration).

Put differently, the predictive model may for example be configured to estimate (predict), based on the concentration measure, an estimated size (or estimated reference size) of the inhibition zone at the reference concentration from a size (e.g., the determined size) of the inhibition zone at the sample concentration. Thereby, the predictive model may for example allow for determining a size of the inhibition zone had the susceptibility testing been performed at the reference concentration instead of at the sample concentration.

Additionally or alternatively, the predictive model may for example also be configured to estimate (predict), based on the concentration measure, an estimated size of the inhibition zone at the sample concentration from a size (e.g., a classification threshold or breakpoint) of the inhibition zone at the reference concentration. Thereby, the predictive model may for example allow for determining an adjusted threshold (e.g., an adjusted classification threshold or breakpoint) that should be applied (e.g., compared with the determined inhibition zone size) to account for the fact that the susceptibility testing is performed at the sample concentration rather than at the reference concentration (which the non-adjusted classification threshold or breakpoint may be associated with).

The reference concentration may be a fixed predetermined concentration. The reference concentration may in particular be a standardized bacterial concentration used as a reference point (or benchmark) in antibiotic susceptibility testing, e.g., a bacterial concentration for which standardized classification thresholds or breakpoints (reference thresholds) for assessing antibiotic susceptibility are available, e.g., from the aforementioned CLSI or EUCAST recommendations. The reference concentration may for example be a McFarland standard such as the McFarland standard No. 0.5 (which roughly corresponds to a concentration of about 1.5 x 10⁸ colony-forming units (CFU) per milliliter) or any one of the McFarland standards No. 1.0, 2.0, 3.0 and 4.0.

By using the predictive model for assessing susceptibility, the method according to the present disclosure may account for variations in bacterial concentration in (e.g., non-standardized) samples. This may allow for a more accurate determination of antibiotic susceptibility while eliminating the need for bacterial isolation and inoculum standardization, which are typically required in conventional methods. This makes the diagnostic process faster and more efficient, particularly in resource-limited settings where time and expertise are scarce, while at the same time reducing the risk of misclassifying bacteria as susceptible or resistant.

Assessing the antibiotic susceptibility of the sample may comprise estimating an estimated size of the inhibition zone at the reference concentration of bacteria from the determined size of the inhibition zone (and from the determined concentration measure) using the predictive model, e.g., to obtain an estimate for a size of the inhibition zone that would be expected if the susceptibility testing had instead been performed at the reference concentration (e.g., as in a conventional susceptibility test performed at a defined bacterial concentration). The antibiotic susceptibility of the sample may be assessed based on the estimated size of the inhibition zone at the reference concentration, for example by comparing the estimated size of the inhibition zone at the reference concentration with a predetermined threshold, e.g., a standardized classification threshold or breakpoint (which, optionally, may additionally take into account the type of antibiotic and/or the bacterial species, i.e., may be an antibiotic- and/or species-specific threshold).

Additionally or alternatively, assessing the antibiotic susceptibility of the sample may comprise estimating an estimated size of the inhibition zone at the sample concentration of bacteria from a size of the inhibition zone, in particular a predetermined threshold for the inhibition zone size, at the reference concentration (and from the determined concentration measure) using the predictive model. This may for example be used to obtain an estimate for an adjusted threshold for the size of the inhibition zone that accounts for the fact that the susceptibility testing is performed at the sample concentration rather than at the reference concentration (that the predetermined threshold, which may, e.g., a standardized classification threshold or breakpoint, may be associated with). The antibiotic susceptibility of the sample may be assessed based on the determined size and the estimated size of the inhibition zone, for example by comparing the determined size of the inhibition zone with the adjusted threshold.

The predictive model may for example be or comprise any statistical, machine-learning (e.g., neural-network) based and/or other mathematical predictive model (e.g., classifier or estimator) relating the inhibition zone sizes associated with the sample concentration and the reference concentration to each other based on the concentration measure. The predictive model may have been obtained (e.g., trained) prior to execution or as part of the method according to the present disclosure, for example based on a set of training data containing inhibition zone sizes determined at different sample concentrations (but for otherwise identical samples and conditions, e.g., a same species of bacteria and/or a same antibiotic), for example at different levels of dilution of a same sample.

The predictive model may for example be or comprise a statistical regression model, in particular a linear regression model and/or a non-linear regression model (polynomial regression model). The statistical regression model may be a mathematical model that models (e.g., estimates) the functional relationship (or dependency) between the inhibition zone sizes associated with the sample and reference concentrations, e.g., provides a mathematical function (with one or more adjustable fit parameters) mapping the inhibition zone size associated with the sample concentration (independent variable x) to the inhibition zone size associated with the reference concentration (dependent variable y(x)) or vice-versa. In case of a linear regression model, said mathematical function may be a linear function (e.g., y = a · x + b), whereas in case of a non-linear regression model said mathematical function may additionally contain one or more higher-order terms (e.g., at least one or both of a quadratic/second-order term x² and a third-order term x³). The statistical regression model may be obtained by regression analysis, e.g., curve fitting techniques such as least squares.

The size of the inhibition zone may be determined using a disk diffusion test. The disk diffusion test may comprise arranging an antibiotic sample (e.g., an antibiotic-impregnated carrier such as a paper disk) on and/or in the substrate, wherein the antibiotic sample may be disk-shaped without, however, being limited to this shape. The disk diffusion test may be performed following the Kirby-Bauer protocol or other established disk diffusion test protocols such as the Oxford penicillin cup test or Etest (Epsilometer test). In other examples, the antibiotic may be applied by other means, for example in liquid and/or solid form.

The sample may be exposed to the antibiotic for, e.g., between 1 hour and 72 hours, in some examples between 6 hours and 48 hours, in one example between 18 hours and 24 hours (e.g., for a predetermined amount of time within one of the aforementioned ranges). The method may comprise incubating the sample, for example after one or both of applying the sample to the substrate and exposing the substate to the antibiotic (e.g., while the substrate is being exposed to the antibiotic).

As mentioned above, the size of the inhibition zone may be determined following an established standard such as the CLSI or EUCAST recommendations. Preferably, however, the size of the inhibition zone is determined taking into account the sample concentration, for example by measuring the size of the inhibition zone using different measurement protocols depending on the concentration measure. Thereby, a more reliable determination may be achieved, in particular at concentrations that are lower and in some examples substantially lower than the defined concentration used in the established standards.

For example, the size of the inhibition zone may be measured by averaging distances between respective pairs of opposing colonies (i.e., diameters of the inhibition zone) at a plurality of locations (e.g., at two, three, four, five or more locations) distributed around an antibiotic sample arranged on and/or in the substrate. This measurement protocol may, e.g., be used in case the sample concentration characterized by the concentration measure lies within a first range (e.g., an intermediate range) of concentrations below the reference concentration. A pair of opposing colonies as used herein may refer to a pair of colonies that are arranged on opposite sides of the antibiotic sample (e.g., rotated by 180° or approximately 180° about the antibiotic sample with respect to each other) and adjacent to (e.g., abutting) an edge of the inhibition zone.

In another example, the size of the inhibition zone may be measured by measuring a distance between the antibiotic sample arranged on and/or in the substrate and the colony of bacteria closest to the antibiotic sample. This measurement protocol may, e.g., be used in case the sample concentration characterized by the concentration measure lies within a second range (e.g., low range) of concentrations below the reference concentration. The measured distance may be doubled, e.g., to determine a diameter of the inhibition zone. The colony of bacteria closest to the antibiotic sample may for example the closest colony to the antibiotic sample meeting a size threshold, for example having at least a predetermined size or a predetermined number of bacteria (e.g., being visibly discernible).

Both measurement protocols may be used in combination, i.e., measuring the inhibition zone size based on the distances between respective pairs of opposing colonies if the concentration measure is within the first range and measuring the inhibition zone size based on the closest colony if the concentration measure is within the second range. The second range of concentrations (e.g., an upper bound thereof) may be below the first range of concentrations (e.g. a lower bound thereof). The first range may for example be a range of intermediate concentrations (below the reference concentration), whereas the second range may for example be a range of low concentrations (below the intermediate concentrations). The first range of concentrations may for example correspond to sample concentrations between 10³ CFU/ml and 10⁷ CFU/ml, in some examples between 10⁴ CFU/ml and 10⁶ CFU/ml, in one example between 10⁴ CFU/ml and 10⁵ CFU/ml and in one example between 10⁵ CFU/ml and 10⁶ CFU/m. The second range of concentrations may for example correspond to sample concentrations below the lower bound of the first range, e.g., less than 10⁵ CFU/ml, less than 10⁴ CFU/ml or less than 10³ CFU/ml.

Additionally or alternatively, the size of the inhibition zone may be determined following an established standard such as the CLSI or EUCAST recommendations (e.g., by measuring, after incubation, the diameter of the inhibition zone, i.e., the clear area around the disk where bacterial growth has been prevented), for example in case the sample concentration characterized by the concentration measure lies within a third range of concentrations above the first and/or second ranges. The third range may for example comprise and, optionally, extend (e.g., be centered) around the reference concentration (e.g., 0.5 McFarland). The third range of concentrations may for example correspond to sample concentrations above the upper bound of the first range, e.g., more than 10⁵ CFU/ml, 10⁶ CFU/ml or 10⁷ CFU/ml.

In some examples, the method may comprise diluting the bacterial sample (e.g., as a whole or an aliquot thereof), in particular prior to applying the sample to the substrate. The bacterial sample may for example be diluted such that the sample concentration is below a predetermined threshold (e.g., below 10⁵ CFU/ml). This may, e.g., be advantageous in case the concentration measure is to be determined from the sample/aliquot that is applied to the substrate, e.g., from an image of the sample on the substrate, as reliably determining the bacterial concentration on the substrate may be challenging in case of high bacterial concentrations.

The bacterial sample is not particularly limited and may be any (e.g., liquid and/or solid) sample containing bacteria. In a preferred embodiment, the sample is a patient specimen, e.g., a sample obtained from the body of a human or animal subject (which may also be referred to as a patient herein even though the subject does not necessarily have to suffer from any medical condition). The patient specimen may for example be (e.g., consist of) urine, sputum or other respiratory tract samples, blood or fractions thereof, bile fluid, an abscess discharge, a punctate, cerebrospinal fluid or combinations thereof.

The size of the inhibition zone may be determined by direct susceptibility testing of the bacterial sample (e.g., the patient specimen). As used herein, direct susceptibility testing may refer to antibiotic susceptibility testing that does not involve at least one, and preferably does not involve both, of isolating bacteria from the sample (e.g., separating the bacteria from other constituents of the sample) and preparing the sample to a defined (e.g., standardized) bacterial concentration. Rather, the patient specimen (e.g., as extracted from the patient) may be applied directly to the substrate without any intermediate sample preparation steps. The concentration of bacteria in the sample may thus not be controlled and as a result may vary substantially depending on patient and/or sample conditions. This is, however, compensated and accounted for by determining the concentration measured and using the predictive model for assessing the susceptibility of the sample. Direct susceptibility testing may preserve the bacterial phenotype (unlike when performing sub-culturing) and may thus provide additional insights and more in-vivo-like results.

The determination of the concentration measure is not particularly limited and may be performed using any method for determining concentrations of bacteria known in the art. The concentration measure may for example be determined optically, e.g., by measuring a turbidity or an optical density and/or by image- (e.g., microscopy-) and/or flow-cytometry-based counting techniques. The inhibition zone size and the concentration measure may be determined from a same aliquot or from different aliquots of the sample.

In some examples, the concentration measure may be determined from the sample (or aliquot thereof) that is applied to the substrate, either prior to, during or after exposure to the antibiotic. The concentration measure may for example be determined from one or more images, in particular microscopic images, of the sample on the substrate. Said one or more images may be the same image(s) that the size of the inhibition zone is determined from. The concentration measure may for example be determined based on a density of bacteria on the substrate (e.g., a number, density and/or size of colonies) and/or a distribution of bacteria on the substrate (e.g., a size, a shape and/or a homogeneity of the distribution of bacteria on the substrate). The concentration measure may be determined using a computer vision technique e.g., a computer-implemented algorithm for processing and/or interpreting visual data, in particular a machine-learning based (e.g., neural network based) image classifier.

In other examples, a first aliquot of the sample is applied to the substrate for determining the size of the inhibition zone, whereas the concentration measure is determined from a second aliquot of the sample (different from the first aliquot). The second aliquot may be incubated on a second substrate. The second substrate may be embodied differently from the first substrate. For example, the first substrate may be or comprise a Mueller-Hinton agar whereas the second substrate may be or comprise a lysogeny broth (LB) agar. Using a second substrate may for example allow for performing the determination of the inhibition zone size and the concentration measure in parallel, in particular in case the later additional involves the identification of the species of bacteria.

The method may comprise identifying a species of bacteria in the sample. The species of bacteria in the sample may for example be identified by mass spectroscopy such as matrix-assisted laser desorption/ionization (MALDI) mass spectrometry and in particular MALDI time-of-flight (TOF) mass spectrometry. Additionally or alternatively, the species of bacteria may, e.g., be identified by gene sequencing or any other technique for identifying a species of bacteria known in the art. Identifying the species of bacteria may comprise isolating bacteria from the sample (e.g., prior to or after applying the sample to a substrate and/or prior to or after incubation).

The species of bacteria may be taken into account when assessing the susceptibility of the sample. For example, the predictive model may be configured to associate a size of the inhibition zone at the sample concentration with a size of the inhibition zone at a reference concentration of bacteria and/or vice-versa based on the concentration measure and the species of bacteria (i.e., taking the species of bacteria as a further input (e.g., independent variable) in addition to the concentration measure). This may for example be achieved by training the predictive model on species-specific training data, e.g., a set of training data containing inhibition zone sizes determined selectively at different sample concentrations and for different species of bacteria. Thereby, species-dependent variations of the relation between the inhibition zone sizes at different concentrations may be accounted for.

Additionally or alternatively, the species of bacteria may also be taken into account in other ways, in particular by using a species-specific threshold for assessing (e.g., classifying) the susceptibility of the sample. This may allow for further improving the accuracy of the determination of antibiotic susceptibility. In certain cases (e.g., certain combinations of species and antibiotic), however, classification thresholds may not vary much between different species of bacteria and thus the identification of the species of bacteria may be omitted to reduced diagnostic processing time or save resources. This for example applies in case of an antibiotic selected from fosfomycin, nitrofurantoin, mecillinam and combinations thereof and bacteria selected from E. coli, K. pneumoniae, P. mirabilis, Enterobacter complex, C. koseri, P. aeruginosa, S. saprophyticus, S. aureus, E. faecalis and combinations thereof (which are the most common uropathogens).

The species may be identified from a same aliquot or from a different aliquot of the sample as used for the determination of the inhibition zone size and/or the determination of the concentration measure. The species identification may for example be performed using the (same) second aliquot as used for determining the concentration measure (which may, e.g., be incubated on a second substrate). In another example, the first aliquot may be used for the determination of the inhibition zone size as well as the concentration measure and the second aliquot may be used for the species identification (e.g., on a second substrate or in suspension). This may allow for performing the determination of the inhibition zone size and the concentration measure as well as the species identification in parallel.

In some examples, the concentration dependence of the inhibition zone size may be accounted for in other ways instead of (or in addition to) using the predictive model. For example, susceptibility of the sample may be assessed using concentration-specific classification thresholds (which, optionally, may also be antibiotic- and/or species-specific). The concentration-specific classification thresholds may for example have been determined beforehand (similar to the training of the predictive model), e.g., by measuring inhibition zone sizes as different concentrations for bacteria of known susceptibility (e.g., determined in parallel at the reference concentration such as 0.5 McFarland using standardized classification thresholds). The concentration-specific classification thresholds may for example be determined by optimizing accuracy of the resulting susceptibility classification based on the known susceptibility of the bacteria. The concentration-specific classification thresholds may be stored in a database and accessed for assessing the susceptibility of the sample. In some examples, susceptibility of the sample may be assessed using interpolated concentration-specific classification thresholds, which may, e.g., be interpolated (for example by weighted averaging) from a set of concentration-specific classification thresholds from the database, each of which may associated with one particular concentrations. In other examples, each concentration-specific classification threshold may be associated with a respective range of the concentration measure or sample concentration and the appropriate classification threshold may then be selected based on the determined concentration measure.

According to a second aspect, the present disclosure provides a computer program comprising machine-readable instructions which, when executed by a processing unit, cause the processing unit to execute a method for antibiotic susceptibility testing of a bacterial sample according to any one of the embodiments descried herein.

The computer program may be stored on (or provided as) an (e.g., non-transitory) machine-readable medium and/or may be stored in a memory of the apparatus according to the present disclosure (e.g., in a (volatile or non-volatile) memory of the controller thereof).

According to a third aspect, the present disclosure provides an apparatus for antibiotic susceptibility testing of a bacterial sample. The apparatus comprises a sample handler configured to apply the sample to a substrate and expose the sample on the substrate to an antibiotic. The apparatus further comprises an imaging unit configured to image the sample on the substrate. The apparatus further comprises a controller that is configured to control the sample handler and the imaging unit and is configured to execute, using the sample handler and the imaging unit, a method for antibiotic susceptibility testing of a bacterial sample of the first aspect according to any one of the embodiments described herein.

The sample handler may be configured to extract the sample (or an aliquot thereof) from a receptacle such as a sample tube containing the sample. The sample handler may be configured to split the sample into two or more aliquots, e.g., at least the first and second aliquots mentioned above. The sample handler may be configured to apply a first aliquot to a (first) substrate for determining the inhibition zone size and a second aliquot to a second substrate, e.g., for species identification and/or determining the concentration measure. The sample handler may comprise a pipetting device (e.g., a pipetting robot) and/or a microfluidic system, for example for applying the sample and/or the antibiotic to the (first) substrate and, optionally, the second substrate.

The imaging unit may comprise an image sensor such as a CCD or CMOS chip configured to record images of the sample on the first substrate and, optionally, the second substrate. The imaging unit may be embodied as a camera, which in addition to the image sensor may, e.g., comprise optics such as an objective (e.g., for magnification). In some examples, the imaging unit may be a microscopic imaging unit configured to record microscopic images of the sample on the first substrate and, optionally, the second substrate.

The controller may be embodied in hardware, software or a combination thereof. The controller may for example comprise one or more processing units and memory storing instructions for execution by the one or more processing units to provide the functionality described herein. The controller may execute and, optionally, store (e.g., in the memory) a computer program according the second aspect of the present invention.

The controller is configured to determine a size of an inhibition zone that is formed when the sample is applied to a substrate and the sample on the substrate is exposed to an antibiotic, e.g., as described above for the method according to the first aspect. The controller is further configured to determine a concentration measure characterizing a sample concentration of bacteria in the sample, e.g., as described above for the method according to the first aspect. The controller is further configured to assess susceptibility of the sample to the antibiotic using a predictive model configured to relate, based on the concentration measure, a size of the inhibition zone associated with the sample concentration to a size of the inhibition zone associated with a reference concentration of bacteria and/or vice-versa, e.g., as described above for the method according to the first aspect.

The controller is configured to control the sample handler and the imaging unit to execute the method for antibiotic susceptibility testing of the bacterial sample. For this, the controller may be configured to provide suitable control signals, e.g., electrical and/or optical control signals to the sample handler and the imaging unit. The controller may for example be configured to control the sample handler to apply the sample to the first substrate and, optionally, the second substrate and to expose the sample on the (first) substrate to the antibiotic (e.g., by arranging an antibiotic sample on and/or in the substrate). The controller may be configured to control the imaging unit to take one or more images of the sample on the first substrate and, optionally, the second substrate.

### LIST OF FIGURES

In the following, a detailed description of the present disclosure and examples thereof is given with reference to the figures. The figures show schematic illustrations of
Fig. 1: an apparatus for antibiotic susceptibility testing of a bacterial sample according to an example of the present disclosure;
Fig. 2: a flow chart of a method for antibiotic susceptibility testing of a bacterial sample according to an example of the present disclosure;
Fig. 3a-c: antibiotic susceptibility tests at different sample concentrations of bacteria according to an example of the present disclosure;
Fig. 4a: experimental results showing the concentration dependence of the measured inhibition zone size according to an example of the present disclosure;
Fig. 4b: regression-based predictive models for relating inhibition zone sizes at different concentrations according to an example of the present disclosure; and
Fig. 5: a flow chart of a parallelized method for antibiotic susceptibility testing of a bacterial sample according to an example of the present disclosure.

### DESCRIPTION OF EXAMPLES

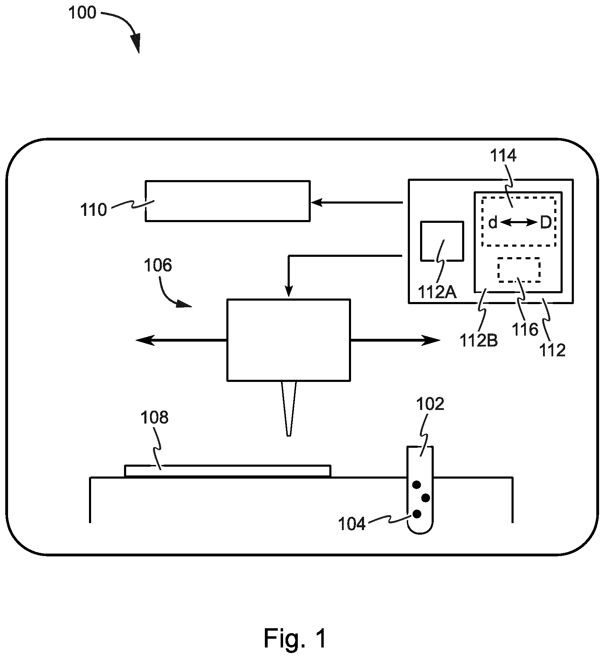

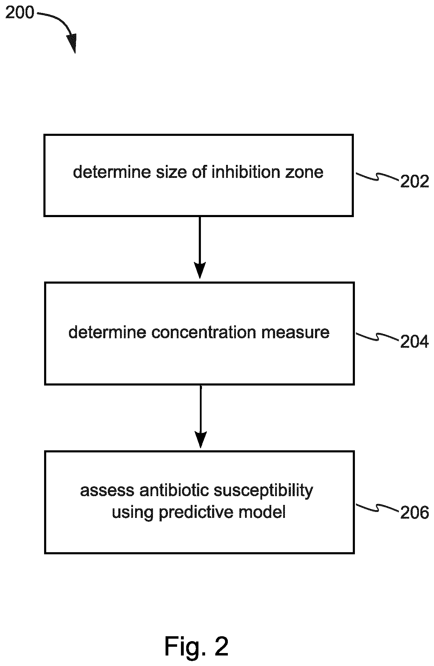

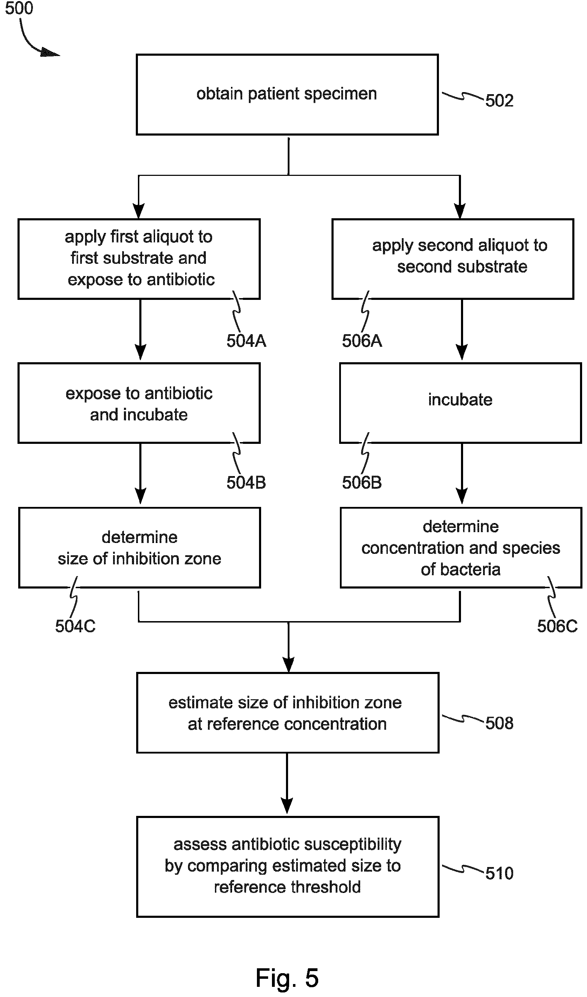

Fig. 1 shows a schematic illustration (not to scale) of an apparatus 100 for antibiotic susceptibility testing of a bacterial sample 102 comprising bacteria 104 according to an example of the present disclosure. The apparatus 100 may be used for executing (and, optionally configured to execute) a method for antibiotic susceptibility testing of a bacterial sample 102 of the first aspect according to any one of the embodiments described herein such as for example one or both of the method 200 of Fig. 2 and the method 500 of Fig. 5 described below.

The apparatus 100 comprises a sample handler 106 configured to apply the sample 102 (e.g., an aliquot thereof) to a substrate 108, e.g., as described below for method 200 and/or step 504A of method 500. The sample handler 106 may for example be configured to apply the sample 102 to the substrate 108 by spreading the sample 102, preferably homogeneously, on a top surface of the substrate 108. The sample handler 106 may for example be embodied as or comprise an automated pipetting device, e.g., a pipetting robot.

The substrate 108 is not particularly limited and may be any substrate (e.g., plate or matrix) suitable for sustaining and, optionally, culturing and/or incubating, bacteria 104 from the sample 102 and for performing antibiotic susceptibility testing thereon. The substrate 108 may comprise or consist of a cell-culture medium. The substrate 108 may in particular be an agar substrate, for example a lysogeny broth (LB) agar substrate or a Mueller-Hinton agar substrate.

The sample handler 106 is further configured to expose the sample 102 on the substrate 108 to an antibiotic, e.g., as described below for method 200 and/or step 504B of method 500. The sample handler 106 may for example be configured to place an antibiotic sample (not shown) such as an antibiotic-impregnated carrier (e.g., an antibiotic-impregnated paper disk) onto the substrate 108, e.g., as described below with reference to Figs. 3a-c.

The apparatus 100 further comprises an imaging unit 110 such as a camera and/or a microscope that is configured to take images of the sample 102 on the substrate 108, e.g., for determination of the inhibition zone size and/or the concentration measure.

The apparatus 100 also comprises a controller 112 that is configured to control the sample handler 106 and the imaging unit 110 by providing suitable control signals thereto. The controller 112 is configured to execute, using the sample handler 106 and the imaging unit 110, a method for antibiotic susceptibility testing of a bacterial sample according to any one of the embodiments described herein. The controller 112 may in particular be configured to execute one or both of the method 200 and the method 500 at least in part or in their entirety.

The controller 112 may be embodied in hardware, software or a combination thereof. In the example of Fig. 1, the controller 112 comprises a processing unit 112A, which may for example be or comprise a central processing unit (CPU), a graphics processing unit (GPU), an application-specific integrated circuit (ASIC), a field-programmable gate array (FPGA), a programmable logic array (PLA), a microcontroller or another processing device. The controller 112 further comprises a memory 112B such as a non-volatile memory (e.g., a flash memory) and/or a volatile memory (e.g., a random-access memory). In one example, the controller 112 may be a computer configured to control the apparatus 100, in particular the sample handler 106 and the imaging unit 110.

The memory 112B stores a predictive model 114 that is configured to relate, based on a concentration measure, a size of the inhibition zone associated with a sample concentration to a size of the inhibition zone associated with a reference concentration of bacteria and/or vice-versa, e.g., as described below for method 200 and/or 500. The memory 112B further stores a computer program 116 with instructions for execution by the processing unit 112A to provide the functionality described herein. The computer program 116 may be a computer program according to the second aspect of the present disclosure. The computer program 116, when executed by the processing unit 112A, may cause the processing unit 112A to execute some or all steps of a method for antibiotic susceptibility testing of a bacterial sample according to any one of the embodiments described herein, in particular some or all steps of any one of methods 200 and 500. The predictive model 114 may be provided separate from or as part of the computer program 116 (i.e., the computer program 116 may or may not comprise the predictive model 114).

The apparatus 100 may further comprise (or be arranged in) a temperature-controlled incubation chamber, e.g., for incubating the sample 102 and/or the substrate 108 at a fixed temperature.

Fig. 2 shows a flow chart of a method 200 for antibiotic susceptibility testing of a bacterial sample according to an example of the present disclosure. The method 200 may be executed using (and, optionally, by) an apparatus for antibiotic susceptibility testing of a bacterial sample of the third aspect of the present disclosure according to any one of the embodiments described herein, for example the apparatus 100 of Fig. 1, which is used as a non-limited example for illustration purposes in the following. This is, however, not intended to be limiting in any way and the method 200 may also be executed using a different apparatus according to the third aspect of the present disclosure or also with apparatus or devices different from the apparatus according to the third aspect of the present disclosure. Furthermore, the method 200 is not limited to the order of execution implied by the flow chart in Fig. 2. As far as technically feasible, the steps of method 200 may be executed in an arbitrary order and may also be executed simultaneously at least in part, for example steps 202 and 204. Some or all steps of method 200 may be executed by the controller 112 of the apparatus 100 using the sample handler 106 and/or the imaging unit 110, e.g., by executing the computer program 116 with the processing unit 112A.

The method 200 comprises, in step 202, determining a size of an inhibition zone that is formed when the sample 102 is applied to the substrate 108 and the sample 102 on the substrate 108 is exposed to an antibiotic, e.g., using the sample handler 106. In some examples, the method 200 may comprise applying the sample 102 to the substrate 108 and exposing the sample 102 to the antibiotic, e.g., as in step 504A of method 500 described below. In other examples, the method 200 may not comprise these steps but may, e.g., start with an image of the sample 102 on the substrate 108 taken after the sample 102 was exposed to the antibiotic (i.e., the steps of applying the sample 102 to the substrate 108 and exposing the sample 102 to the antibiotic may be performed beforehand and separate from execution of the method 200). The sample 102, the substrate 108 and the antibiotic may for example be embodied as described in the following with reference to Figs. 3a-c and/or as described below for method 500 of Fig. 5.

The size of the inhibition zone may for example be determined using a disk diffusion test. This is shown in Figs. 3a-c, which schematically illustrate disk diffusion tests at different sample concentrations of bacteria (e.g., different concentrations prior to application of the sample 102 to the substrate 108) according to an example of the present disclosure. In particular, Fig. 3a schematically illustrates an exemplary disk diffusion test at a reference concentration (e.g., an 0.5 McFarland standard), Fig. 3b at an intermediate concentration (e.g., at 10⁵ CFU/ml) and Fig. 3c at a low concentration (e.g., at 10³ CFU/ml).

The disk diffusion tests comprise applying the sample 102 with the bacteria 104 to the substrate 108 (which may, e.g., be disk-shaped as illustrated in Figs. 3a-c), for example by spreading the sample 102, preferably homogeneously, on a top surface of the substrate 108. An antibiotic sample 300, which may, e.g., be an antibiotic-impregnated and preferably disk-shaped carrier such as a paper disk, is placed at the center of the substate 108. The substrate 108 with the bacteria 104 and the antibiotic sample 300 is incubated, e.g., at 37 °C, for a predetermined incubation time, e.g., between 20 and 24 hours. At the reference concentration, the substrate 108 (following incubation and outside the inhibition zone 302) may essentially be covered homogeneously by a film of bacteria 104 with no individual colonies discernible as illustrated in Fig. 3a. At the intermediate concentration, there may be areas with little or no bacteria in between a more or less homogeneous bacterial film or a plurality of colonies, which may or may not overlap at least partially, e.g., as illustrated in Fig. 3b. At the low concentration, there may only be a number of colonies, which are spatially separated from each other, e.g., as illustrated in Fig. 3c.

The antibiotic may diffuse from the antibiotic sample 300 and may kill susceptible bacteria 104 and/or inhibit growth thereof, leading to the formation of an inhibition zone (also referred to as inhibition halo) 302 around the antibiotic sample 300. The size of the inhibition zone 302 may be indicative of the susceptibility of the bacteria 104 from the sample 102 to the respective antibiotic. Following the incubation, the substrate 108 (with the bacteria 104 thereon) may be imaged, e.g., using the imaging unit 110, for determining the size, e.g., the diameter or radius, of the inhibition zone 302. This may yield images similar to the schematic illustrations of Figs. 3a-c, which may, e.g., be analyzed by the controller 112, e.g., using computer vision techniques, and/or by a user (e.g., using or operating the controller 112 and/or the apparatus 100).

The size of the inhibition zone 302 may vary depending on the concentration of bacteria 104. In particular, the inhibition zone 302 may be larger at low concentrations as in Fig. 3c and smaller at high concentrations as in Fig. 3a. For example, the inhibition zone 302 may have a (reference) diameter D at the reference concentration in Fig. 3a, a diameter d₁ at the intermediate concentration in Fig. 3b and a diameter d₂ at the low concentration in Fig. 3c, wherein D < d₁ < d₂.

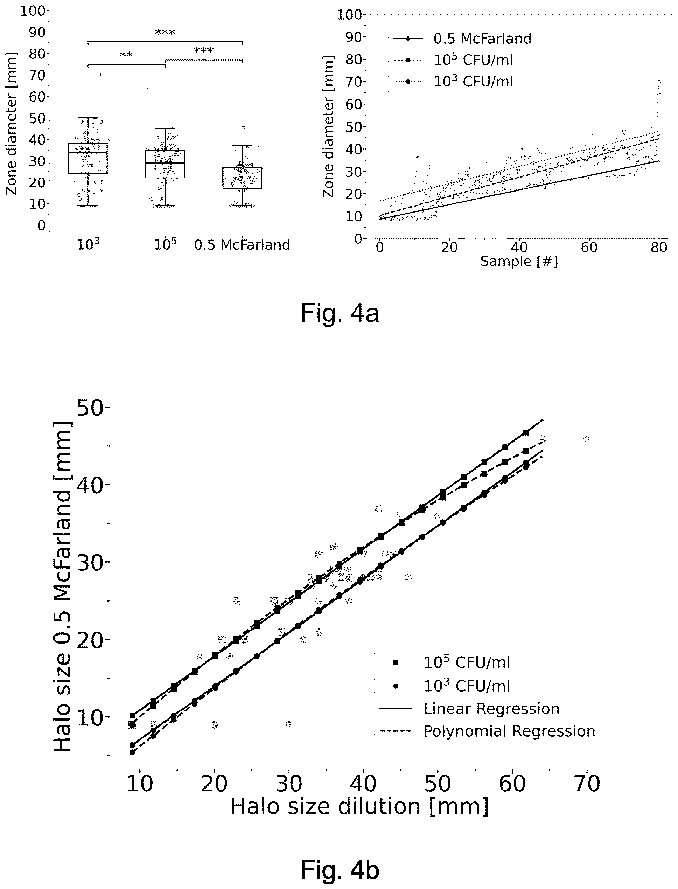

This is illustrated by the experimental results in Fig. 4a showing the concentration dependence of the measured inhibition zone size ("zone diameter") according to an example of the present disclosure. For this, 0.5 McFarland suspensions of 81 clinical urine samples were diluted to 10⁵ CFU/ml and 10³ CFU/ml. The suspensions at the different concentrations were assessed using three different antibiotics, namely fosfomycin, nitrofurantoin, and mecillinam (n=243). Samples were analyzed based on their inhibition zones, revealing a consistent pattern in zone sizes across different concentrations but with significant differences in inhibition zone diameters between the 0.5 McFarland suspensions and their dilutions (p < 0.001 for 0.5 McFarland vs. 10⁵ CFU/ml and 0.5 McFarland vs. 10³ CFU/ml, p < 0.01 for 10⁵ CFU/ml vs. 10³ CFU/ml). The inhibition zone size increased as bacterial concentration decreased from high to intermediate to low CFU/ml.

The diameter D of the inhibition zone 302 at the reference concentration in Fig. 3a may for example be measured following the standard EUCAST protocol. This protocol may, however, not be appropriate at intermediate and/or low concentrations as in Figs. 3b and 3c. Instead, the size d₁ of the inhibition zone 302 at intermediate concentrations may for example be measured by averaging distances between respective pairs (e.g., for three or more pairs) of opposing colonies arranged on opposite sides of the antibiotic sample 300 (and adjacent to, e.g., or abutting the outer edge of the inhibition zone 302) as illustrated in Fig. 3b. The size d₂ of the inhibition zone 302 at low concentrations may for example be measured by measuring a distance between the antibiotic sample 300 and the colony of bacteria 104 that is closest to the antibiotic sample 300 (and doubling said distance/radius to obtain the diameter d₂ as illustrated in Fig. 3c).

The method 200 further comprises, in step 204, determining a concentration measure characterizing a sample concentration of bacteria 104 in the sample 102. The concentration measure may for example be (or be indicative of or quantify) the concentration of bacteria 104 on the substrate 108 (following incubation), e.g., outside of the inhibition zone 302. This concentration may in turn depend on and thus characterize (e.g., be indicative of or allow for quantifying) the concentration of bacteria in the sample 102 prior to application to the substrate 108 and/or prior to exposure to the antibiotic and incubation (as an example of a sample concentration). The concentration measure may for example be determined from a density and/or a distribution of the bacteria 104 on the substrate 108, for example by determining a coverage ratio of the substrate 108, e.g., a fraction of the surface area of the substrate 108 covered by bacteria 104. The concentration measure may be determined from the same image of the substrate 108 used for determining the inhibition zone size, for example by the controller 112 (e.g., using computer vision techniques), and/or by a user. In other examples, the concentration measure may be determined by a separate workflow, e.g., from a second aliquot of the sample 102, for example as in steps 506A-506C of the method 500 described below.

Finally, in step 206, the susceptibility of the sample 102 (i.e., of the bacteria 104 contained therein) to the antibiotic is assessed based on the size of the inhibition zone 302 determined in step 202 and additionally taking into account the concentration measure determined in step 204. The susceptibility is assessed using a predictive model such as the predictive model 114 stored in the memory 112B of the controller 112.

The predictive model 114 is configured to relate, based on the concentration measure, a size of the inhibition zone 302 associated with the sample concentration to a size of the inhibition zone 302 associated with a reference concentration of bacteria 104 and/or vice-versa. Thereby, the predictive model 114 for example allows for converting the inhibition zone diameters d₁ and d₂ at the intermediate and low concentration, respectively, to an estimated reference diameter D that would be expected had the corresponding susceptibility test been performed at the (e.g., standardized) reference concentration instead. In this way, varying concentrations in the sample 102 (e.g., as a result of not suspending the sample to a defined bacterial concentration) may be accounted for.

This is illustrated by way of example in Fig. 4b, which depicts the inhibition zone size ("halo size") measured in susceptibility tests of clinical isolates tested against fosfomycin at a reference concentration of 0.5 McFarland versus the inhibition zone size measured in susceptibility tests of the same samples diluted to 10⁵ CFU/ml and 10³ CFU/ml, respectively. The experimental data from Fig. 4b again confirms the significant concentration dependence of the inhibition zone size already observed in Fig. 4a (i.e., the halo size at 10³ CFU/ml is larger than at 10⁵ CFU/ml, which in turn is larger than the halo size at the reference concentration). The reference concentration may for example be a standardized concentration for susceptibility testing, e.g., as recommended by EUCAST.

Data as in Fig. 4b (and/or Fig. 4a) may be used as training data for training the predictive model. The predictive model may for example be a linear or non-linear (e.g., quadratic/second-order) statistical regression model and may be fitted to the training data (e.g., separately for each non-reference concentration and, optionally, separately for each antibiotic and/or bacterial species). The trained (fitted) predictive models are illustrated by the solid (linear) and dashed lines (non-linear/polynomial) in Fig. 4b.

For assessing the antibiotic susceptibility, the inhibition zone size determined in step 202 (e.g., the diameter d₁ or d₂) may be converted to the estimated reference size D as expected at (predicted for) the reference concentration using the predictive model 114. The estimated reference size D may then be compared against one or more standardized classification thresholds (e.g., EUCAST classification threshold(s)) associated with the reference concentration. The reference concentration should accordingly be chosen to match the standardized concentration for which the classification thresholds are provided. Based on the classification threshold(s), the sample 102 (i.e., the bacteria 104 contained therein) may then be classified as susceptible or resistant (or, optionally, partially susceptible). In some examples, the inhibition zone size conversion may be performed the other way around, i.e., instead of converting the determined inhibition zone size to an estimated reference size at the reference concentration, the classification threshold may instead be converted, using the predictive model, to an adjusted classification threshold for the sample concentration, against which the determined inhibition zone size may then be compared.

Fig. 5 shows a flow chart of a method 500 for antibiotic susceptibility testing of a bacterial sample according to another example of the present disclosure. The method 500 may be executed using (and, optionally, by) an apparatus for antibiotic susceptibility testing of a bacterial sample of the third aspect of the present disclosure according to any one of the embodiments described herein, for example the apparatus 100 of Fig. 1, which is used as a non-limited example for illustration purposes in the following. This is, however, not intended to be limiting in any way and the method 500 may also be executed using a different apparatus according to the third aspect of the present disclosure or also with apparatus or devices different from the apparatus according to the third aspect of the present disclosure. Furthermore, the method 500 is not limited to the order of execution implied by the flow chart in Fig. 5. As far as technically feasible, the steps of method 500 may be executed in an arbitrary order and may also be executed simultaneously at least in part (in particular steps 504A-C on one hand and steps 506A-C on the other hand). Some or all steps of method 500 may be executed by the controller 112 of the apparatus 100 using the sample handler 106 and/or the imaging unit 110, e.g., by executing the computer program 116 with the processing unit 112A.

The method 500 is similar to the method 200 of Fig. 2 described above but differs from the method 200 in that the method 500 additionally involves sample handling (e.g., steps 502, 504A/B and 506A/B) and determines the inhibition zone size and the concentration measure from two different aliquots of the sample 102 that are applied to two different substrates 108 and processed in two separate and preferably parallelized workflows (steps 504A-C and steps 506A-C).

The method 500 comprises, in step 502, obtaining the sample 102 (e.g., receiving the sample 102 from outside the apparatus 100 and, optionally, also extracting the sample 102 from a patient). In the example of Fig. 5, the sample 102 is a patient specimen that was obtained (or is obtained as part of step 502) directly from the body of a patient, for example a urine sample from a patient with a urinary tract infection. The sample 102 may contain bacteria 104 such as, e.g., E. coli, K. pneumoniae, P. mirabilis, Enterobacter complex, C. koseri, P. aeruginosa, S. saprophyticus, S. aureus, E. faecalis, and combinations thereof.

Step 502 further comprises splitting the sample 102 into two or more aliquots, namely at least a first aliquot for determination of the inhibition zone size in steps 504A-C and a second aliquot for determination of one or both of the concentration measure and the species of bacteria in the sample 102 in steps 506A-C. Using the two aliquots, the determination of the inhibition zone size in steps 504A-C and the determination of the concentration measure and/or the species of bacteria in steps 506A-C are then preferably performed in parallel (i.e., simultaneously at least in part), which may drastically reduce diagnostic processing time as compared to conventional methods.

In step 504A, the first aliquot is applied to a first substrate 108, e.g., using the sample handler 106. The first aliquot may be applied to the first substrate by spreading the first aliquot, preferably homogeneously, on a top surface of the first substrate 108. The first aliquot is preferably applied to the first substrate 108 without any intermediate processing steps therebetween, in particular without isolation of bacteria 104 and suspension to a defined concentration, i.e., by directly applying the patient specimen to the substrate 108 (direct susceptibility testing).

In step 504B, the first aliquot on the first substrate 108 is exposed to an antibiotic (e.g., using sample handler 106) and incubated (e.g., at 37 °C) in the presence of the antibiotic, for example by means of a disk diffusion test as described with reference to Figs. 3a-c above. The antibiotic is not particularly limited and may, e.g., be any antibiotic as used for treating bacterial infections, in particular urinary tract infections. The antibiotic may for example be or comprise one or more of fosfomycin, nitrofurantoin, mecillinam, trimethoprim, cephalosporins, ciprofloxacin, levofloxacin, piperacillin/tazobactam, gentamicin, amikacin, imipenem/cilas-tatin, meropenem, and plazomicin (which are the most common antibiotics for treating urinary tract infections).

In step 504C, the size of the inhibition zone 302 that is formed on the first substrate 108 following exposure to the antibiotic and incubation is determined, e.g., as described above for step 202 of the method 200.

In step 506A, the second aliquot is applied to a second substrate 108, e.g., using the sample handler 106. The second substrate 108 may be different from the first substrate 108. For example, the first substrate 108 may be or comprise a Mueller-Hinton agar substrate whereas the second substrate 108 may be or comprise a lysogeny broth (LB) agar substrate. Application of the second aliquot may be similar to the application of the first aliquot in step 504A. However, the application of the second aliquot in step 506A may additionally comprise one or more intermediate processing steps prior to or after arranging the second aliquot on the second substrate 108, in particular isolating the bacteria 104 (e.g., from other constituents of the sample 102/the second aliquot). In other examples, bacterial isolation may be performed later on, e.g., following incubation in step 506B.

In step 506B, the second aliquot on the second substrate 108 is incubated, e.g., similar to step 504B (but without exposure to the antibiotic). Finally, in step 506C, the concentration of bacteria 104 on the second substrate 108 (as an example of a concentration measure) is determined, e.g., similar to step 204 of method 200 (i.e., by imaging of the bacteria 104 on the substrate 108 expect that the determination of the concentration measure is now performed with a separate aliquot on a separate substrate 108). In other examples, the concentration of bacteria 104 on the second substrate 108 (or in the sample or the second aliquot without application to a second substrate 108) may be determined by other means, e.g., in suspension and/or by measuring a turbidity or optical density (noting that for samples containing other particles, substances or the like that could influence turbidity or optical density, as may, e.g., be the case for urine, measuring the concentration of bacteria 104 on a (first or second) substrate 108 may yield more reliable results than turbidity or optical density measurements) . In yet other examples, the concentration measure may be determined using the first aliquot on the first substrate 108, e.g., as in step 204 of method 200.

Additionally or alternatively, step 506C may also comprise determining a species of bacteria 104, e.g., using MALDI-TOF mass spectrometry, for example to distinguish between the aforementioned bacterial species and, optionally, determine a fraction of the respective species among the total bacterial load of the sample 102.

In step 508, an estimated size of the inhibition zone 302 at a reference concentration of bacteria 104 is estimated from the inhibition zone size determined in step 504C using the predictive model, e.g., similar to step 206 of method 200. For estimating the size at the reference concentration, the concentration measure and, optionally, the species of bacteria 104 determined in step 506C and/or the type of antibiotic are taken into account (e.g., by using a predictive model trained with concentration- and, optionally species- and/or antibiotic-specific training data or separate predictive models for each combination of concentration (e.g., concentration range) and, optionally, species and/or antibiotic).

Finally, in step 510, antibiotic susceptibility of the bacteria 104 in the sample 102 is assed by comparing the estimated size at the reference concentration to a standardized classification threshold (reference threshold), e.g., as described above for step 206 of method 200. The reference threshold may be a species- and/or antibiotic specific threshold and may, e.g., be selected based on the species of bacteria 104 determined in step 506C.

The examples of the present disclosure disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

- 100 -: apparatus for antibiotic susceptibility testing of a bacterial sample
- 102 -: sample/bacterial sample
- 104 -: bacteria
- 106 -: sample handler
- 108 -: substrate
- 110 -: imaging unit
- 112 -: controller
- 112A: processing unit
- 112B -: memory
- 114 -: predictive model
- 116 -: computer program

- 200 -: method for antibiotic susceptibility testing of a bacterial sample

- 300 -: antibiotic sample
- 302 -: inhibition zone
- D -: size of inhibition zone at reference concentration
- d₁, d₂ -: size of inhibition zone at intermediate and low sample concentration, respectively

- 500 -: method for antibiotic susceptibility testing of a bacterial sample

## Claims

1. A method (200, 500) for antibiotic susceptibility testing of a bacterial sample (102), the method (200, 500) comprising:
determining a size (d) of an inhibition zone (302) that is formed when the sample (102) is applied to a substrate (108) and the sample (102) on the substrate (108) is exposed to an antibiotic (300);
determining a concentration measure characterizing a sample concentration of bacteria (104) in the sample (102); and
assessing a susceptibility of the sample (102) to said antibiotic using a predictive model (114) configured to relate, based on the concentration measure, a size (d) of the inhibition zone (302) associated with the sample concentration to a size (D) of the inhibition zone (302) associated with a reference concentration of bacteria (104) and/or vice-versa.

2. The method (200, 500) of claim 1, wherein assessing the susceptibility of the sample (102) to said antibiotic comprises:
estimating an estimated size (D) of the inhibition zone (302) at the reference concentration of bacteria (104) from the determined size (d) of the inhibition zone (302) using the predictive model (114); and
assessing the susceptibility of the sample (102) to the antibiotic based on the estimated size (D) of the inhibition zone (302) at the reference concentration.

3. The method (200, 500) of claim 1 or 2, wherein said predictive model (114) is or comprises a statistical regression model, in particular a linear regression model and/or a non-linear regression model.

4. The method (200, 500) of any one of the preceding claims, wherein the size (d) of the inhibition zone (302) is determined using a disk diffusion test.

5. The method (200, 500) of any one of the preceding claims, wherein the size (d) of the inhibition zone (302) is determined taking into account the sample concentration by measuring the size (d) of the inhibition zone (302) using different measurement protocols depending on the concentration measure.

6. The method (200, 500) of claim 5, wherein the size (d) of the inhibition zone (302) is measured by averaging distances between respective pairs of opposing colonies at a plurality of locations distributed around an antibiotic sample (300) arranged on and/or in the substrate (108) in case the sample concentration **characterized by** the concentration measure lies within a first range of concentrations below the reference concentration.

7. The method (200, 500) of claim 5 or 6, wherein the size (d) of the inhibition zone (302) is measured by measuring a distance between the antibiotic sample (300) arranged on and/or in the substrate (108) and the colony of bacteria (104) closest to the antibiotic sample (300) in case the sample concentration **characterized by** the concentration measure lies within a second range of concentrations below the reference concentration.

8. The method (200, 500) of claim 6 and 7, wherein the second range of concentrations is below the first range of concentrations.

9. The method (200, 500) of any one of the preceding claims, wherein the sample (102) is a patient specimen, in particular urine, and the size (d) of the inhibition zone (302) is determined by direct susceptibility testing of said specimen.

10. The method (200, 500) of any one of the preceding claims, wherein the concentration measure is determined based on a density and/or a distribution of bacteria (104) on the substrate (108), in particular using a computer vision technique.

11. The method (200, 500) of any one of the preceding claims, wherein a first aliquot of the sample (102) is applied to said substrate (108) for determining the size (d) of the inhibition zone (302) and the concentration measure is determined from a second aliquot of the sample (102), in particular a second aliquot of the sample (102) incubated on a second substrate.

12. The method (200, 500) of any one of the preceding claims, further comprising identifying a species of bacteria (104) in the sample (102), wherein said predictive model (114) is configured to associate a size (d) of the inhibition zone (302) at the sample concentration with a size (D) of the inhibition zone (302) at a reference concentration of bacteria (104) and/or vice-versa based on the concentration measure and the species of bacteria (104).

13. The method (200, 500) of claim 11 and 12, wherein the species identification is performed using the second aliquot of the sample (102).

14. A computer program (116) comprising machine-readable instructions which, when executed by a processing unit (112A), cause the processing unit (112A) to execute a method (200, 500) according to any one of claims 1 to 13.

15. An apparatus (100) for antibiotic susceptibility testing of a bacterial sample (102), the apparatus (100) comprising:
a sample handler (106) configured to apply the sample (102) to a substrate (108) and expose the sample (102) on the substrate (108) to an antibiotic (300);
an imaging unit (110) configured to image the sample (102) on the substrate (108); and
a controller (112) configured to control the sample handler (106) and the imaging unit (110) and to execute a method (200, 500) according to any one of claims 1 to 13 using the sample handler (106) and the imaging unit (110).
